# EUROPEAN PATENT APPLICATION

(11) **EP 3 560 473 A1**
(43) Date of publication of application: **30.10.2019**
(21) Application number: 17882730.9
(22) Date of filing: 16.05.2017
(51) Int. Cl.: A61H 3/04, A61G 7/02, A61G 7/018, E04H 1/12

(54) **INTELLIGENT NURSING SYSTEM**

(30) Priority: 23.12.2016 CN 201611204964
(71) Applicant: Ecarelife Technologies Co., Ltd., Beijing 100085 (CN); Luoyang Advanced Manufacturing Industrial R&D Center, Tianjin Research Institute for Advanced Equipment, Tsinghua University, Hi-tech Industry Development Zone Luoyang Henan 471000 (CN)
(72) Inventor: HAN, Zhenghe, Beijing 100084 (CN); LIN, Guan, Beijing 102206 (CN); DING, Qiushi, Beijing 100084 (CN)
(74) Representative: Gulde & Partner
(86) International application number: PCT/CN2017/084486
(87) International publication number: WO 2018/113175

(57) **Abstract**

An intelligent nursing system, comprising at least one or a combination of some of a bed function module (6), a toilet function module (5), a bathing function module (7), a seat function module (2), and a rehabilitation exercise function module (9), as well as a walking and transferring function module (4). Each function module comprises a butt joint (3, 401); the walking and transferring function module (4) and other function modules are configured to be connected by means of the butt joint (3, 401); a user can move by means of the walking and transferring function module (4), and can achieve transferring between the walking and transferring function module (4) and other function modules; the walking and transferring function module (4) further has a lifting capability so as to help the user achieve transferring between the walking and transferring function module (4) and other function modules. The intelligent nursing system enables old people or people with reduced mobility to autonomously complete at least one of nursing activities in daily life, without a nursing staff.

## Description

### TECHNICAL FIELD

Embodiments of the present disclosure relate to an intelligent nursing system.

### BACKGROUND

As the problem of population aging has intensified, China is rapidly entering an ageing society. The primary problem that the aging society faces is how to care for the growing proportion of the elderly. On one hand, the fast pace of modern society makes it difficult to maintain the traditional mode of relying on children to support the elderly. At the same time, the trend of declining birth caused by the one-child policy and the change of the concept of birth also aggravates the pension dilemma of young families in the new era. On the other hand, although it is a common social phenomenon to send the elderly to rest homes or to hire home-based service personnel who stay at home to care for the elderly, the pipeline services of rest homes cannot ensure that every elderly people can live with dignity. At the same time, due to the reduction of the labor force, there is not enough nursing staff that can ensure that the elderly are cared at home. Therefore, relying on modern technology to achieve intelligent nursing service for the elderly is the main direction to solve the problem of old-age care.

### SUMMARY

The embodiments of the present disclosure provide an intelligent nursing system, and the embodiments of the present disclosure can enable a care recipient to complete at least one nursing activity in daily life without the assistance of a nursing staff.

At least one embodiment of the present disclosure provides an intelligent nursing system including: a movement transfer function module; and at least one selected from the group consisting of a bed function module, a toilet bowl function module, a bath function module, a seat function module and a rehabilitation exercise function module. Each function module among the movement transfer function module, the bed function module, the toilet bowl function module, the bath function module, the seat function module and the rehabilitation exercise function module includes a connection joint; and in a situation where two or more than two function modules are configured to cooperate with each other, the two or more than two function modules are configured to be connected with each other through the connection joints of the two or more than two function modules.

At least one embodiment of the present disclosure also provides another intelligent nursing system including: a nursing function module which includes at least one selected from the group consisting of a bed function module, a toilet bowl function module, a bath function module, a seat function module and a rehabilitation exercise function module; and a movement function module configured to move to the nursing function module. Each of the nursing function module and the movement function module includes a connection joint, and the connection joints of the nursing function module and the movement function module are configured to be rigidly connected with each other.

### BRIEF DESCRIPTION OF THE DRAWINGS

In order to clearly illustrate the technical solution of the embodiments of the disclosure, the drawings of the embodiments will be briefly described in the following; it is obvious that the described drawings are only related to some embodiments of the disclosure and thus are not limitative of the disclosure.
FIG. 1 is a schematic diagram illustrating a module composition of an intelligent nursing system provided by the embodiments of the present disclosure;
FIG. 2 is a schematic activity space layout diagram of the intelligent nursing system provided by the embodiments of the present disclosure;
FIG. 3 is a schematic diagram of a movement transfer function module which is in a folded state and is included by the intelligent nursing system provided by the embodiments of the present disclosure;
FIG. 4 is a schematic diagram of the movement transfer function module which is in a manned movement state and is included by the intelligent nursing system provided by the embodiments of the present disclosure;
FIG. 5 is a side view of the movement transfer function module which is in a state of assisting the user to stand and is included by the intelligent nursing system provided by the embodiments of the present disclosure;
FIG. 6 is a top view of the movement transfer function module which is in the state of assisting the user to stand and is included by the intelligent nursing system provided by the embodiments of the present disclosure;
Figures (a) and (b) in FIG. 7 are schematic diagrams of a multi-stage ascending-descending device of the movement transfer function module of the intelligent nursing system provided by the embodiments of the present disclosure;
Figures (a) and (b) in FIG. 8 are schematic diagram of a driven wheel track changing mechanism of the movement transfer function module of the intelligent nursing system provided by the embodiments of the present disclosure;

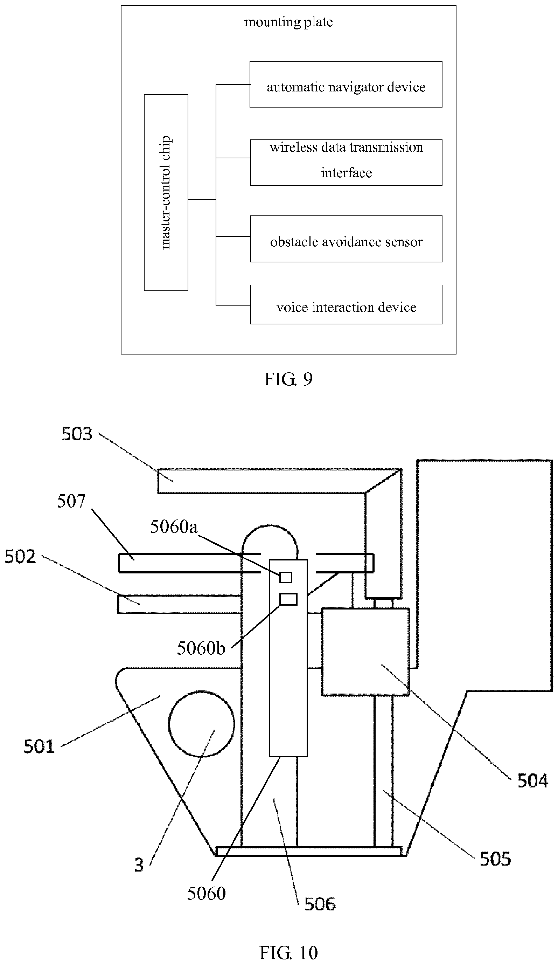

FIG. 9 is a schematic structural view of an automatic navigator device, a wireless data interface, an obstacle avoidance sensor, a voice interaction device and a master-control chip which are at a mounting plate of the movement transfer function module of the intelligent nursing system provided by the embodiments of the present disclosure;
FIG. 10 is a schematic structural view of a toilet bowl function module of the intelligent nursing system provided by the embodiments of the present disclosure;

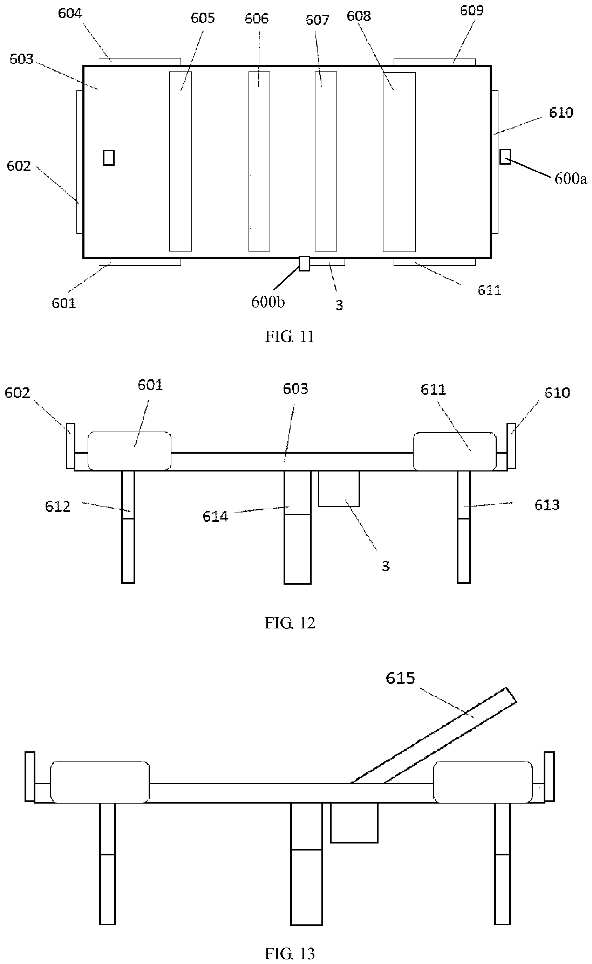

FIG. 11 is a top view of a bed function module of the intelligent nursing system provided by the embodiments of the present disclosure;
FIG. 12 is a side view of the bed function module of the intelligent nursing system provided by the embodiments of the present disclosure;
FIG. 13 is a schematic diagram of the bed function module of the intelligent nursing system provided by the embodiments of the present disclosure in a situation where a user is in a half-lying posture;

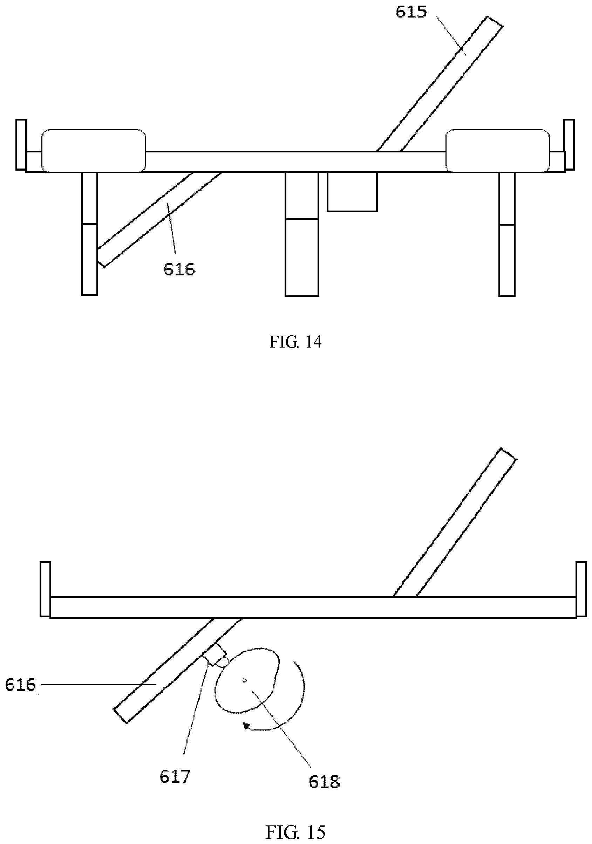

FIG. 14 is a schematic diagram of the bed function module of the intelligent nursing system provided by the embodiments of the present disclosure in a situation where the user is in a sitting posture;
FIG. 15 is a schematic diagram of the bed function module of the intelligent nursing system provided by the embodiments of the present disclosure in a situation where the user performs leg rehabilitation exercise;

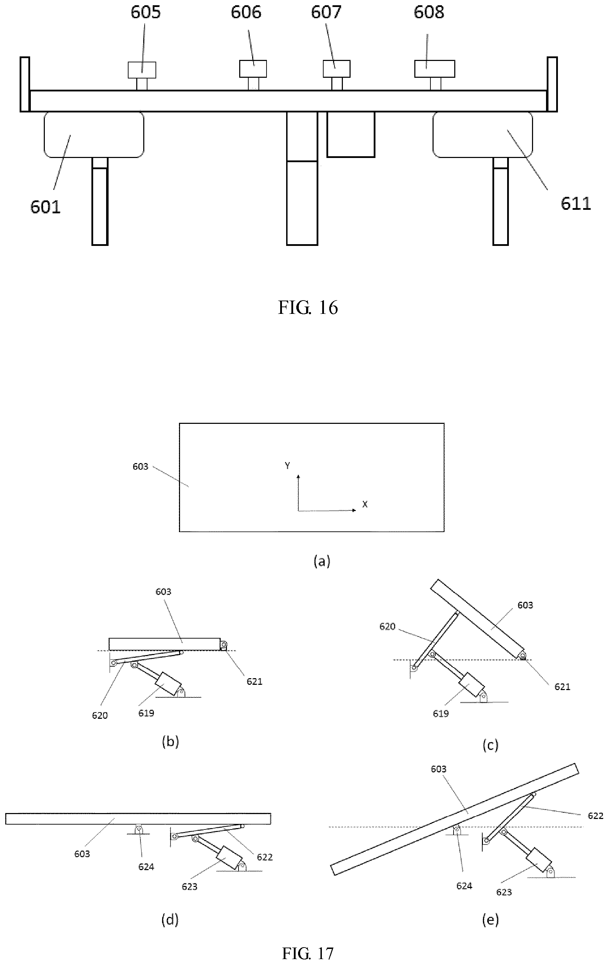

FIG. 16 is a schematic diagram illustrating that a supporting plate of the bed function module of the intelligent nursing system is in a raised state provided by the embodiments of the present disclosure;
Figures (a) to (e) in FIG. 17 are schematic diagrams illustrating that the bed function module of the intelligent nursing system rotates integrally in two mutually perpendicular directions provided by the embodiments of the present disclosure;

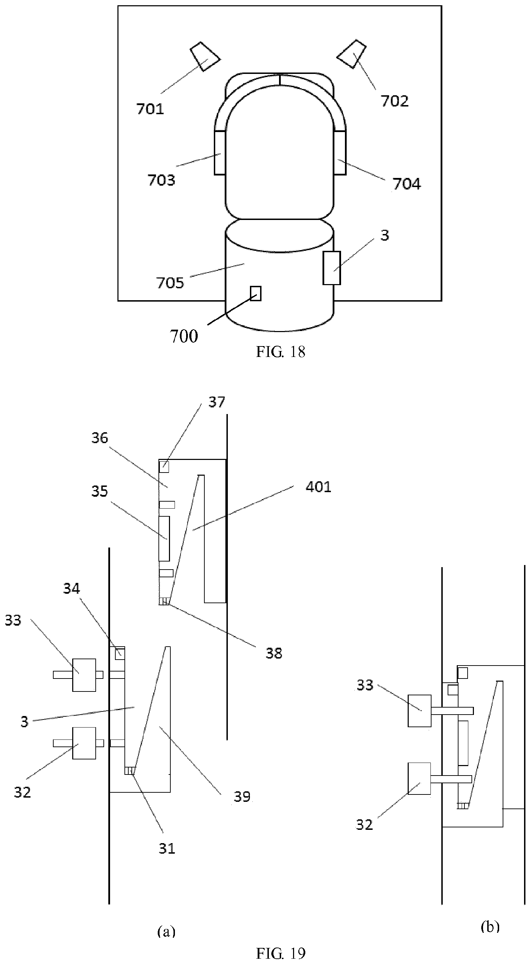

FIG. 18 is a schematic diagram of a bath function module of the intelligent nursing system provided by the embodiments of the present disclosure;
Figures (a) and (b) in FIG. 19 are schematic diagrams of the intelligent nursing system provided by the embodiments of the present disclosure before and after abutment of connection joints;

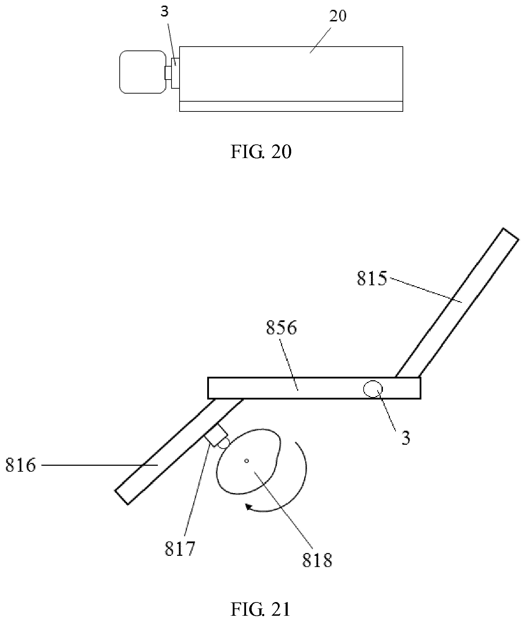

FIG. 20 is a schematic diagram of a seat function module of the intelligent nursing system provided by the embodiments of the present disclosure;
FIG. 21 is a schematic diagram of a rehabilitation exercise function module of the intelligent nursing system provided by the embodiments of the present disclosure; and

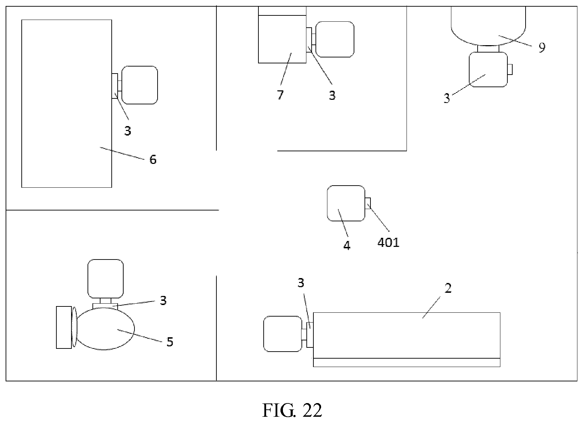

FIG. 22 is a schematic structural diagram of modules of another intelligent nursing system provided by the embodiments of the present disclosure.

### DETAILED DESCRIPTION

In order to make objects, technical details and advantages of the embodiments of the disclosure apparent, the technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

Unless otherwise defined, all the technical and scientific terms used herein have the same meanings as commonly understood by one of ordinary skill in the art to which the present disclosure belongs. The terms "first," "second," etc., which are used in the description and the claims of the present application for disclosure, are not intended to indicate any sequence, amount or importance, but distinguish various components. The terms "comprise," "comprising," "include," "including," etc., are intended to specify that the elements or the objects stated before these terms encompass the elements or the objects and equivalents thereof listed after these terms, but do not preclude the other elements or objects. The phrases "connect", "connected", etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

For the elderly who need to be cared for, the main problem that the daily care faces in a certain age group is: due to aging, the mobility of the leg and the foot is reduced, and it is hard to complete free activities between daily necessities such as a bed, a toilet bowl, a bathtub, a sofa, etc. Although a wheelchair can realize a function of movement between different exercise yards, for example, moving from a bedroom to a toilet, when a care recipient needs to move between the wheelchair and other items, for example, moving from the bed to the wheelchair and from the wheelchair to the toilet, it is difficult to rely on the user per se to complete the action. This is because a relative displacement generates between the wheelchair and the bed or the toilet bowl during the movement of the user, which makes the movement very dangerous and difficult for the elderly, so the movement has to be assisted by a caregiver. For the elderly who have lost their mobility and have to be in bed, they need to rely on the caregiver to carry them so as to carry out necessary movements in the daily life of the elderly. At the same time, people with disabilities who are inconvenient or incapable of moving their leg and foot for various reasons also face the same care need.

At present, there are no reports on the comprehensive daily life nursing system for the elderly or people having difficulty in moving. Common designs and inventions mainly focus on nursing beds or wheelchairs. The nursing beds or the wheelchairs can only partially relieve the workload of nursing staff, and the care recipients still need a lot of manual care in their daily lives. For example, in order to solve the toilet problem of the care recipients, in many designs, a defecation hole is arranged in the bed or the wheelchair. On one hand, this design cannot achieve the function of allowing the care recipient to defecate autonomously, and on the other hand, the requirement for care of most people is living with dignity on the basis of maintaining existing living habits. And to defecate and urinate in the bed or the wheelchair obviously cannot meet this requirement. It is hoped that through an intelligent nursing system, the elderly or the people having difficulty in moving can live independently and with dignity, which provides a better solution for the pension problem that the whole society faces in the future.

The embodiments of the disclosure provides an intelligent nursing system, which can assist the elderly or the people having difficulty in moving to better realize self-care daily life under the condition of unattended care, including posture change, walking, washing, urination and defecation, bathing, and rehabilitation exercise and the like. The system includes a movement transfer function module, a bed function module, a toilet bowl function module, a bath function module, a seat function module, a washing function module and a rehabilitation exercise function module (for example, the system consists of these modules). The movement transfer function module is configured to be able to move towards any one of the remaining function modules. In the intelligent nursing system, these function modules can perform a task separately, or the movement transfer function module can cooperate with any one or more function modules. Each function module in the system is provided with a connection joint (abutting joint). In a situation where two or more function modules work in cooperation, corresponding function modules are connected with each other through the connection joints of the corresponding function modules, and the user can move autonomously (for example, the user relies on upper limbs) between the function modules, or the user can move between the function modules with the aid of the function modules. For example, in a situation where the user moves from the bed function module to the movement transfer function module, the movement is realized through the cooperation of the bed function module and the movement transfer function module. Here, "moving between the function modules" means moving from a position of the movement transfer function module to s position of another function module or from the position of another function module to the position of the movement transfer function module. The connection between the connection joints has sufficient mechanical strength to ensure the required stiffness of the combination of the function modules. For example, the connection joints can realize a data transmission function between the function modules after being connected with each other. In other embodiments of the present disclosure, the connection joints may not transmit data after connection.

For example, in at least one embodiment of the present disclosure, the connection joint (hereinafter referred to as "second connection joint") of each of the bed function module, the toilet bowl function module, the bath function module, the seat function module and the rehabilitation exercise function module and the connection joint (hereinafter referred to as "first connection joint") of the movement transfer function module are configured to be rigidly connected with each other. Due to the rigid connection between the connection joints, when one connection joint is displaced, the other connection joint connected to the one connection joint is not displaced relative to the one connection joint; by fixing the two connection joints respectively on respective function modules, no relative displacement generates between the two function modules, thereby ensuring the safe and smooth movement of the user.

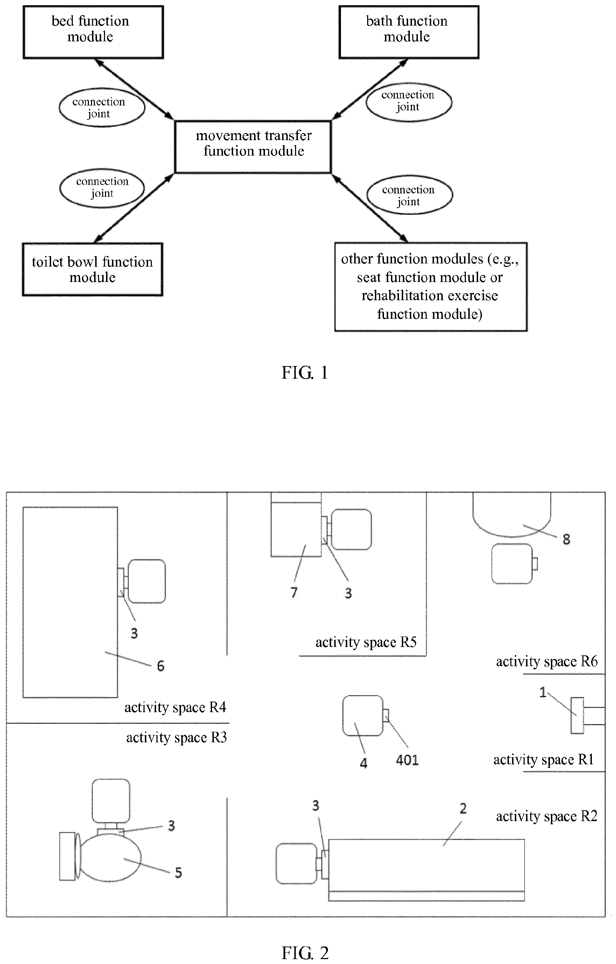

FIG. 1 is a schematic diagram illustrating the module composition of the intelligent nursing system provided by an embodiment of the present disclosure, where arrows in the figure indicate moving directions of the user. The user can move between the movement transfer function module and the bed function module, the bath function module, the toilet bowl function module or other function modules (e.g., the seat function module or the rehabilitation exercise function module or the like), and simultaneously realize the daily life action such as toilet, bathing and indoor activities through the movement of the movement transfer function module. When the user needs to move between the movement transfer function module and any one of the above function modules, the two function modules are rigidly connected with each other through the connection joints to ensure safe and smooth movement of the user.

It should be noted that description is given in FIG. 1 by taking the case that the intelligent nursing system includes the movement transfer function module and other nursing function modules having nursing function as an example. In other embodiments of the present disclosure, the intelligent nursing system not only includes the movement and transfer function but also includes at least one selected from the group consisting of the nursing function modules such as the bed function module, the toilet bowl function module, the bath function module, the seat function module and the rehabilitation exercise function module.

As shown in FIG. 2 which is a space layout diagram of the intelligent nursing system provided by an embodiment of the present disclosure. The reference signs in the figure are respectively: 1. a charging seat; 2. the seat function module (e.g., a sofa function module); 3. the second connection joint; 4. the movement transfer function module; 401. the first connection joint; 5. the toilet bowl function module; 6. the bed function module; 7. the bath function module; 8. the washing function module.

In an embodiment of the present disclosure, different function modules are distributed in different activity spaces, and the movement transfer function module 4 transports the user so that the user can move in the different activity spaces. An activity space R1 represents a functional area in which the movement transfer function module 4 performs an action autonomously. When it is detected that charging is required, the movement transfer function module 4 moves to the activity space R1, and automatic charging is completed by utilization of the charging seat 1. Activity spaces R2 to R5 represent functional areas in which the user needs to move between the movement transfer function module 4 and other function modules. After the movement transfer function module 4 moves to the vicinity of any one of the toilet bowl function module 5, the bed function module 6, the bath function module 8 and the seat function module 2, the rigid connection between the two function modules is realized through the connection between the second connection joint 3 and the first connection joint 401; after the connection is completed, the user can move between the two function modules autonomously, or can achieve the movement by utilization of the carrying function of the movement transfer function module 4. An activity space R6 represents a functional area in which the user does not need to move between the function modules in daily life. The movement transfer function module 4 moves to the vicinity of the washing function module 8; because the user does not need to move between two function modules, washing can be performed after a braking device of the movement function module 4 is adopted for braking.

For instance, in any one of the above embodiments of the present disclosure, a working voltage of each components of the function modules does not exceed 36V during the use of the user.

### First Embodiment

In the intelligent nursing system provided by an embodiment of the present disclosure, for instance, the movement transfer function module can transport the user to a specified destination and is also configured to help the user to transfer from one function module to another function module (for instance, transfer the user from one function module to an upper side of another function module). For instance, the movement transfer function module is also configured to help the user to realize the conversion between a sitting posture and a standing posture. For instance, the movement transfer function module may also be used together with other devices such as a car. When the movement transfer function module is used with other devices, corresponding connection joints can be mounted at the devices as required.

For instance, the movement transfer function module is configured to have a non-manned state and a manned state. For instance, the movement transfer function module is configured to have a folded state and/or a state of assisting the user to stand.

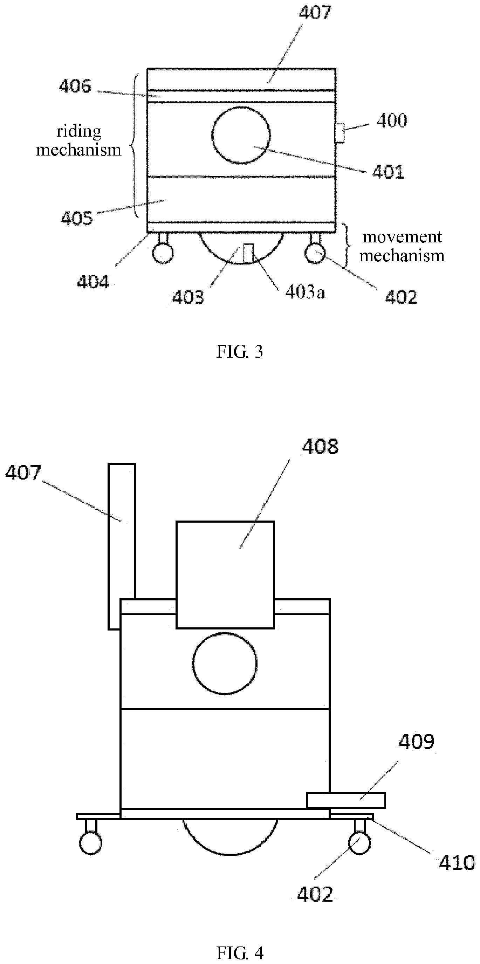

For instance, as shown in FIG. 3 which is a schematic diagram of the movement transfer function module of the intelligent nursing system provided by an embodiment of the present disclosure in the folded (for instance, completely folded) state (one example under the non-manned state), the reference signs in the figure are respectively: 400. an illuminator device; 401. the first connection joint; 402. a driven wheel; 403. a drive wheel; 403a. an electromagnetic brake device; 404. a chassis; 405. a multi-stage ascending-descending device (also referred to as a piecewise ascending-descending device); 406. a cushion; 407. a backrest.

For instance, the first connection joint 401 of the movement transfer function module is disposed on one side of or below the movement transfer function module or at other positions of the movement transfer function module. The movement transfer function module is configured to be connected with other function modules through the first connection joint 401.

For instance, the drive wheel 403 may include a single wheel for drive or includes two wheels which are configured to be driven separately. For instance, the drive wheel 403 includes an electromagnetic brake device 403a.

For instance, the movement transfer function module includes a movement mechanism (movement platform) for realizing a moving function and a riding mechanism (riding platform) for realizing a manned function. The movement mechanism includes the chassis 404, the drive wheel 403, the driven wheels 402 and a driven wheel slide rail (not shown in FIG. 3). The drive wheel 403 is mounted at the chassis 404, and the driven wheels 402 are connected with the chassis 404 through the driven wheel slide rail. The riding mechanism includes the multi-stage ascending-descending device 405, the cushion 406, armrests (not shown in FIG. 3), the backrest 407 and a pedal (not shown in FIG. 3). The multi-stage ascending-descending mechanism 405 is disposed on an upper side of the chassis 403; the cushion 406 is mounted on an upper side of the multi-stage ascending-descending device 405; the backrest 407 is placed on an upper side of the cushion 406 (for instance, the backrest 407 may be placed on the upper side of the cushion 406 and be in a folded state or the backrest 407 may be unfolded); the armrest and the pedal are configured to be able to be folded or unfolded; and under an unfolded state, the armrests are disposed on two sides of the riding mechanism, and the pedal is disposed in the front of the chassis 404 and is higher than an upper surface of the chassis 404. For instance, the illuminator device 400 is also mounted in the front of the riding mechanism of the movement transfer function module.

For instance, under the non-manned state (for instance, the folded state), the armrest and the pedal are folded to the inside of the movement transfer function module, and the backrest is folded to be on the upper side of the cushion.

For instance, under the non-manned state (for instance, the folded state), the movement transfer function module is configured to perform non-manned movement, e.g., automatic charging. For instance, the movement transfer function module is battery-driven and is configured to start an automatic charging program, for example, moving to a position of the charging seat 1 as shown in FIG. 2 for charging, in a situation where the intelligent nursing system detects that the electrical quantity of the battery is reduced to be lower than a certain threshold.

As shown in FIG. 4 which is a schematic diagram of the movement transfer function module of the intelligent nursing system provided by an embodiment of the present disclosure under a manned movement state, the reference signs in the figure are respectively: 407. the backrest; 408. a right armrest; 409. the pedal; 410. the driven wheel slide rail; 402. the driven wheel. For instance, under the manned movement state (for instance, when the user moves from other function module to the movement transfer function module and rides in the sitting posture), the backrest 407, the armrest (for instance, the right armrest 408 and/or a left armrest) and the pedal 409 are in the unfolded state and are respectively disposed at respective functional positions. It should be noted that the left and right armrests can be respectively in the unfolded state or in the folded state independently according to different requirements. For instance, under the manned movement state, the driven wheels 402 connected to the driven wheel slide rail 410 protrude from positions below the chassis to increase a driven wheel track (distance between driven wheels), thereby ensuring the stability of the movement transfer function module.

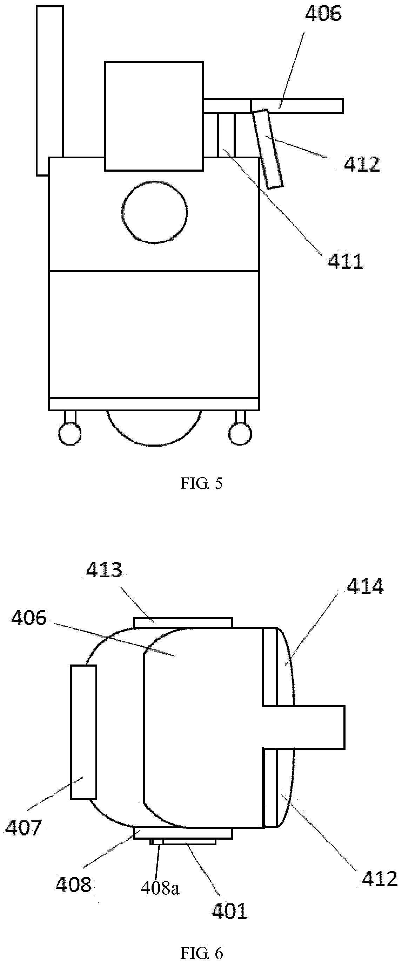

As shown in FIGS. 5 and 6 which are a side view and a top view of the movement transfer function module under a state of assisting the user to stand of the intelligent nursing system provided by an embodiment of the present disclosure, the reference signs in the figure are respectively: 406. the cushion; 411. a cushion support rod; 412. a cushion plate; 413. a left armrest; 414. a cushion plate; 408a. a control handle. For instance, the riding mechanism of the movement transfer function module further includes the cushion plate 412. When the movement transfer function module works under the state of assisting the user to stand, the pedal 409 is retracted. Under the cooperative operation of the multi-stage ascending-descending device 405, the cushion 406 is raised up and protrudes obliquely upward, and the cushion plates 412, 414 on two sides of the front end of the cushion are folded downward to narrow the front end of the cushion, so that the user can change from the sitting posture to a riding posture, thereby facilitating the user to stand. For example, during a process that the movement transfer function module assists the user in standing, the driven wheels 402 can be extended or can be contracted to be below the chassis.

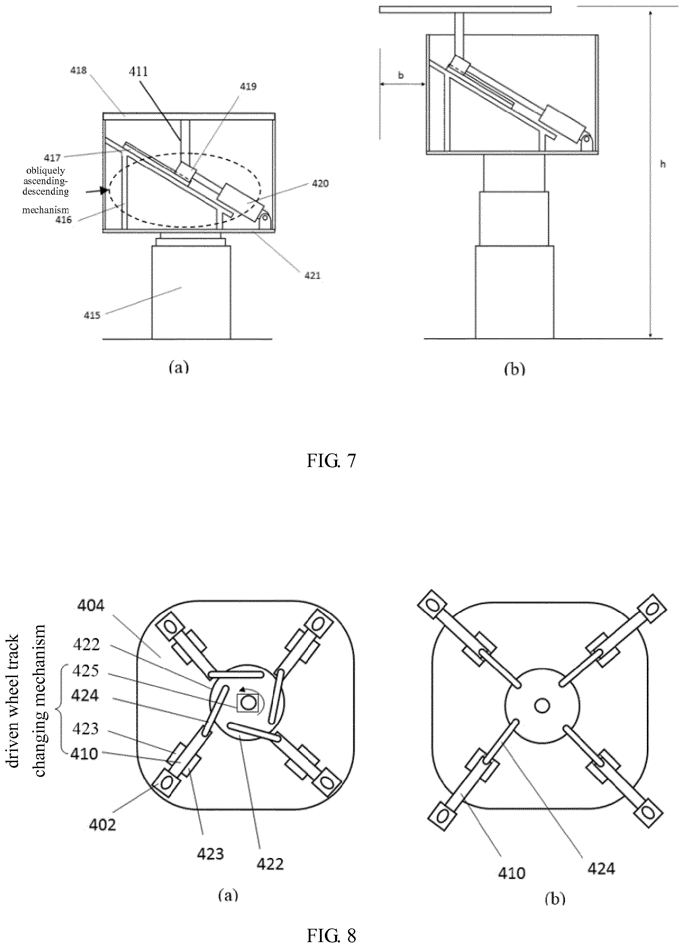

As shown in figures (a) and (b) in FIG. 7 which are schematic diagrams of the multi-stage ascending-descending device of the movement transfer function module of the intelligent nursing system provided by an embodiment of the present disclosure, the reference signs in the figure are respectively: 415. an ascending-descending pillar; 416. an oblique support frame; 417. a guide rail; 418. a cushion base plate; 419. a slider; 420. a push rod; 421. a support frame. For instance, the multi-state ascending-descending system can ascend or descend continuously and adjustably or can ascend or descend uncontinuously but adjustably. For instance, as shown in the figure, the multi-stage ascending-descending device includes the cushion support rod 411, the support frame 421, the ascending-descending pillar 415 that can ascend or descend in a vertical direction, and an obliquely ascending-descending mechanism that can ascend or descend in an obliquely upward direction. The obliquely ascending-descending mechanism includes the oblique support frame 416, the guide rail 417, the slider 419 and the push rod 420, and the slider 419 is connected with the cushion support rod 411. The support frame 421 and the obliquely ascending-descending mechanism can ascend or descend (for instance, ascend or descend continuously and adjustably) in the vertical direction when driven by the ascending-descending pillar 415, and both the armrests 408 and 413 and the backrest are connected with the support frame 421 and can simultaneously ascend or descend together with the cushion when driven by the ascending-descending pillar 415. The push rod 420 is configured to push the slider 419 to move towards the obliquely upward direction along the guide rail 417 while driving the cushion 406 to move obliquely upward, so as to achieve the function of helping the user to stand. For instance, when the push rod 420 moves, the ascending-descending pillar 415 may also simultaneously drive the armrests and the backrest to ascend or descend, so that the movement transfer function module can move to a position that is most suitable for the user to stand.

In FIG. 7(b), b represents a distance at which the obliquely ascending-descending mechanism can protrude forward, the distance is related to a size of the obliquely ascending-descending mechanism, and h represents a height of the cushion from the ground during lifting. For example, in the embodiment of the present disclosure, the minimum distance from the upper surface of the cushion to the ground is not greater than 400 mm, and a ratio of the maximum distance, which is from the upper surface of the cushion to the ground, to the minimum distance is not greater than 2.5.

For instance, the riding mechanism can also be configured to be converted into a transfer mechanism for transferring the user; in a situation where the riding mechanism is used as a transfer platform, the cushion and the armrest in the riding mechanism can be converted into a transfer mechanical arm to transfer the user and to enable the user to move between the movement transfer function module and other modules.

For instance, the number of the driven wheels is variable, and can vary from one to many. The specific quantity is determined according to actual needs. For instance, the number of the driven wheels may be 4. For instance, the movement mechanism includes a plurality of driven wheels, and the driven wheel track can be changed during the movement of the movement transfer function module.

For instance, the movement mechanism may include a driven wheel track changing mechanism. For instance, as shown in (a) and (b) in FIG. 8 which are schematic diagrams of the driven wheel track changing mechanism of the movement transfer function module of the intelligent nursing system provided by an embodiment of the present disclosure, the reference signs in the figure are respectively: 422. a rotating disc; 423. a slider; 424. a connecting rod. The figure shows the track changing mechanism when the movement mechanism includes four driven wheels. For instance, the driven wheel track changing mechanism includes driven wheel slide rails 410, the sliders 423, the connecting rods 424, the rotating disc 422 and a motor 425. The driven wheel 402 is fixed at the driven wheel slider rail 410, and the driven wheel slide rail 410 is connected with the rotating disc 422 through the connecting rod 424. When the rotating disc 422 rotates around a central axis (as shown by a circle in the center of the rotating disc in the figure) of the rotating disc 422 when driven by the motor 425, the connecting rod 424 is adopted to push the driven wheel slide rail 410 to be contracted or extended in the direction of the slider 423 to change the driven wheel track. For instance, when the rotating disc 422 rotates counterclockwise around the central axis of the rotating disc 422 when driven by the motor 425, the connecting rod 424 is adopted to push the driven wheel slide rail 410 to protrude out of the chassis 404 in the direction of the slider 423 and simultaneously drive the driven wheel 402 to move outward; and when the rotating disc 422 rotates clockwise, the driven wheel slide rail 410 is driven to be contracted towards the inside of the rotating disc 422 along the slider 423, and the driven wheel 402 is simultaneously driven to move inward, so as to achieve the function of changing the driven wheel track. During the manned movement of the movement transfer function module, the driven wheels 402 protrude out of the chassis 422, so as to ensure the movement stability of the movement transfer function module.

For instance, the movement manner of the movement transfer function module includes a manual control manner and/or an automatic control manner.

For instance, the movement transfer function module adopts, for instance, the control handle 408a as shown in FIG. 6 to control under the manual control manner, and the control handle 408a is mounted at the armrest on one side (for instance, mounted at the right armrest 408, as shown in FIG. 6).

For instance, as shown in FIG. 9, under the automatic control manner, path guidance is realized through an automatic navigator device mounted at the movement transfer function module; the movement transfer function module is provided with a wireless data transmission interface to execute a command by means of wireless remote control; the movement transfer function module is provided with an obstacle avoidance sensor to achieve a function of stop or bypass when encountering an obstacle; and the movement transfer function module is provided with a voice interaction device to provide a voice prompt before or after the abutment and/or transfer action, the voice interaction device has the functions of voice recognition and voice positioning, and can determine a position of the user according to voice information sent by the user. When the user issues a voice command, the voice interaction device can accept and recognize the user's voice signal and determine the position of the user; the automatic navigator device guides the movement transfer function module to the position of the user; and then the user is transferred to the movement transfer function module.

For instance, the movement transfer function module further includes a master-control chip; a mounting plate is disposed on the upper side of the chassis; the automatic navigator device, the wireless data transmission interface, the obstacle avoidance sensor, the voice interaction device and the master-control chip are mounted at the mounting plate; and the control handle, the automatic navigator device, the wireless data transmission interface, the obstacle avoidance sensor and the voice interaction device are respectively connected with the master-control chip.

For instance, the automatic navigator device may adopt a magnetic navigator, a laser navigator, an inertial navigator, or the like.

### Second Embodiment

As shown in FIG. 10 which is a schematic structural view of the toilet bowl function module of the intelligent nursing system provided by an embodiment of the present disclosure, the reference signs in the figure are respectively: 3. the second connection joint; 501. a toilet bowl; 502. an auxiliary heightening cushion for toilet bowl; 503. a toilet bowl armrest; 504. a cushion ascending-descending slider; 505. an ascending-descending guide rail; 506. an auxiliary trouser removal device; 507. a cleaning and drier device (for instance, a smart cleaning toilet lid or a smart cleaning toilet mat). The toilet bowl function module includes the toilet bowl 501, the auxiliary heightening cushion 502, the smart cleaning toilet lid 507, the toilet bowl armrest 503, the auxiliary trouser removal device (for instance, an automatic trouser removal mechanism) 506 and the connection joint 3. For instance, the connection joint 3 is mounted on a side of the toilet bowl 501 and can be connected with the movement transfer function module. The auxiliary heightening cushion 502 is mounted on an upper side of the toilet bowl 501 and can adjust the hip height of the user using the toilet bowl. For instance, the auxiliary heightening cushion 502 of the toilet bowl can move up and down along the ascending-descending guide rail 505 when driven by the cushion ascending-descending slider 504, so as to adjust the height of the auxiliary heightening cushion to facilitate the elderly to stand after toilet. For instance, the auxiliary heightening cushion 502 also has a cushion ring heating function. The armrests 503 are disposed on two sides of the toilet bowl 501 and can be folded and can ascend or descend. The auxiliary trouser removal device 506 is disposed beside the toilet bowl 501, and a main structure of the auxiliary trouser removal device 506 includes auxiliary trouser removal mechanical arms 5060 respectively disposed on two sides of the toilet bowl 501. A connecting buckle 5060a is disposed at an end portion of the auxiliary trouser removal mechanical arm and is configured to be connected with the user's waist of trousers. The end portion of the auxiliary trouser removal mechanical arm 5060 can move up or down along the direction of user's thigh to complete trouser removal or trouser wearing action through the cooperative movement of the auxiliary trouser removal mechanical arms 5060 on the two sides of the toilet bowl 501. The motion direction, the motion speed and the motion distance of the auxiliary trouser removal mechanical arms 5060 can be predetermined, or the motion of the auxiliary trouser removal mechanical arms 5060 can be manually controlled. A pulling force sensor 5060b can be mounted at the auxiliary trouser removal mechanical arm 5060. When the pulling force exceeds a certain value, the auxiliary trouser removal mechanical arm 5060 stops motion. At the same time, the cleaning and drier device 507 such as the smart cleaning toilet lid mounted at the toilet bowl is configured to help the user to clean and dry after toilet.

The process of transferring the user from the movement transfer function module to the toilet bowl function module through the cooperative work of the movement transfer function module and the toilet bowl function module is as follows:
1. The user issues an instruction;
2. The movement transfer function module moves close to the toilet bowl function module and is connected with the toilet bowl through the connection joint; and
3. The height of the seat of the movement transfer function module is adjusted to be consistent with the height of the cushion of the toilet bowl.

Two methods are included in subsequent steps according to user status:
(1) The user can sit up by himself/herself and the upper limbs of the user can basically function:
   1. The armrest which is included by the movement transfer function module and is on a side close to the toilet bowl and the armrest which is included by the toilet bowl function module and is on a side close to the movement transfer function module are retracted;
   2. The user translates (moves horizontally) from the movement transfer function module to the toilet bowl cushion;
   3. The armrest which is included by the toilet bowl and is on the side close to the movement transfer function module is raised up; the user leaves the toilet bowl cushion under the assistance of the armrest of the toilet bowl; and the auxiliary trouser removal device completes an trouser removal action;
   4. After the toilet bowl is used, the user leaves the toilet bowl cushion under the assistance of the armrest of the toilet bowl, and completes a trouser wearing action with the help of the auxiliary trouser removal device, and then translates from the toilet bowl to the seat of the movement transfer function module;
   5. The seat of the movement transfer function module returns to a height suitable for walking/movement;
   6. The movement transfer function module is separated from the toilet bowl function module and goes to the next destination.
(2) The user cannot sit up on his/her own and the upper limbs of the user are insufficient in function:
   1. The armrest which is included by the movement transfer function module and is on the side close to the toilet bowl and the armrest which is included by the toilet bowl and is on the side close to the movement transfer function module are retracted;
   2. Two mechanical arms of the movement transfer function module protrudes; one mechanical arm is on the back of the user, and meanwhile, a guardrail protrudes and encircles the chest and the underarm of the user to form protection; and the other mechanical arm is in the lower limb part of the user, and lifts up the user and transfers the user to the top of the toilet bowl;
   3. The mechanical arm on the back of the user cooperates with the front guardrail to take up the main weight of the user, and meanwhile, cooperates with the mechanical arm supporting the lower limb, so that the upper body of the user is in a nearly vertical state; the auxiliary trouser removal device completes the trouser removal action; and the mechanical arm places the user on the toilet bowl;
   4. After the toilet bowl is used, the two mechanical arms of the movement transfer function module protrudes and lift up the user according to the method described in the foregoing steps; the auxiliary trouser removal device completes the trouser wearing action; and the mechanical arms put the user on the seat of the movement transfer function module;
   5. The seat of the movement transfer function module returns to the height suitable for walking/movement;
   6. The movement transfer function module is separated from the toilet bowl function module and goes to the next destination.

The execution of each step above can be realized through human-computer interaction between the user and the device if necessary.

### Third Embodiment

For instance, in an embodiment of the present disclosure, the bed function module is a combined structure and mainly includes a bed surface, a supporting plate, armrests, baffle plates and the connection joint. The supporting plate is mounted in the middle portion of the bed surface. The armrests are disposed on both sides of the bed surface. The baffle plates are disposed at a head end and a tail end of the bed surface. Both the armrests and the baffle plates can be adjusted in height and can be lowered to positions below the plane of the bed surface.

As shown in FIGS. 11 and 12 which are a top view and a side view of the bed function module of the intelligent nursing system provided by an embodiment of the present disclosure, the reference signs in the figure are respectively: 3. the second connection joint; 601, 604, 609 and 611. side armrests of the bed function module; 602 and 610. baffle plates at the head end and the tail end of the bed; 603. the bed surface; 605, 606, 607 and 608. ascending-descending supporting plates; 612 and 613. auxiliary support rods; 614. an ascending-descending pillar. The second connection joint 3 mounted on the side can be connected with other function module, for instance, connected with the movement transfer function module. The side armrests 601, 604, 609 and 611 mainly have the function of protecting the user and avoiding fall, and meanwhile, also have the ascending-descending function to facilitate the movement of the user between the bed and other objects, for instance, facilitating the movement of the user between the bed function module and other function modules. The ascending-descending supporting plates 605, 606, 607 and 608 can lift up the user and allow the user's body to leave the bed surface 603 to facilitate the transfer of the user. The ascending-descending pillar 614 can control the ascending-descending of the bed surface 603. For instance, control buttons 600a are disposed at end portions of the head end and the tail end of the bed function module and can control the motion of the components of the bed function module; and/or a data transmission interface 600b is also disposed at the bed function module and can be connected with a smart machine (e.g., a smart mobile phone) for remote control of the motion of the bed function module.

For example, the bed surface is in a split joint form and includes a back plate, a hip plate and a leg plate. By adjusting the height and the angle of different components of the bed surface 603, the user can change between flat-lying, half-lying and sitting postures. For example, the height of the bed surface can be adjustable.

FIG. 13 is a schematic diagram of the bed function module of the intelligent nursing system provided by an embodiment of the present disclosure when the user is in the half-lying posture. In the figure, 615 refers to the back plate. The back plate 615 is rotatable and can stop at any angle, so that the user can change from the flat-lying posture to the half-lying posture, which is convenient for the user to sit up and move.

For instance, the bed function module has massage and rehabilitation exercise functions, and a part which is included by the bed surface and is configured for placing the user's leg (i.e., a leg plate) can swing continuously to drive the lower limb of the care recipient to move.

FIG. 14 is a schematic diagram of the bed function module of the intelligent nursing system provided by an embodiment of the present disclosure when the user is in the sitting posture. In the figure, 615 refers to the back plate; 656 refers to the hip plate; and 616 refers to the leg plate (e.g., a lower leg plate). The leg plate can swing up and down continuously to assist the user in leg rehabilitation.

As shown in FIG. 15 which is a schematic diagram of the bed function module of the intelligent nursing system provided by an embodiment of the present disclosure when the user performs leg rehabilitation exercise, the reference sign 617 in the figure refers to a roller, and the reference sign 618 refers to a cam mechanism. When the user is in the sitting posture on bed, by the movement of the cam mechanism 618 mounted below the bed, the roller 617 which is in contact with the cam mechanism 618 drives the leg plate 616 to continuously swing up and down around a fixed end of the leg plate, thereby achieving the rehabilitation exercise function of the user's leg.

For instance, the supporting plates 605-608 are placed side by side and are respectively disposed below the back, the hip and the leg of the user in flat-lying posture, and at least partial supporting plates of the bed function module can ascend or descend to lift up the user to form a certain spacing between the user and the bed surface, thereby facilitating the transfer of the user.

As shown in FIG. 16 which is a schematic diagram of the bed function module of the intelligent nursing system provided by an embodiment of the present disclosure when the supporting plates are in a raised state, the armrests 601 and 611 on the side of the bed in the figure are lowered to positions below the bed surface, and the supporting plates 605, 606, 607 and 608 are raised from the bed surface to allow the user's body to leave the bed surface, thereby facilitating the transfer of the user through the mechanical arms of the movement transfer function module.

For instance, the bed function module includes two rotation axes which mutually form 90°, one axis is parallel to the body direction when the user is flat-lying down, and the other axis is perpendicular to the body direction when the user is flat-lying down; the bed surface 603 and the two rotation axes are connected with each other through a rotating mechanism; and the bed surface 603 can be integrally rotatable around the two rotation axes.

As shown in (a)-(e) in FIG. 17 which are schematic diagrams of the bed function module of the intelligent nursing system provided by an embodiment of the present disclosure when integrally rotating in two mutually perpendicular directions, the reference signs in the figure are: 619. a push rod; 620. a connecting rod; 621. a hinged terminal; 622. another connecting rod; 623. another push rod; 624. another hinged terminal. Figure (a) in FIG. 17 shows an X axis and a Y axis which are perpendicular to each other in the bed surface. Figures (b) and (c) in FIG. 17 are schematic diagrams illustrating the rotation of the bed surface around the X axis, in which the hinged terminal 621 is a fixed terminal (one example of the rotating mechanism), namely the position of the hinged terminal 621 is fixed, and the push rod 619 pushes the connecting rod 620 to move, so as to push the bed surface 603 to rotate around the hinged terminal 621, thereby realizing the rotation of the bed surface around the X axis. Figures (d) and (e) in FIG. 17 are schematic diagrams illustrating the rotation of the bed surface around the Y axis, in which the push rod 623 pushes the connecting rod 622 to move, so as to push the bed surface 603 to rotate around the hinged terminal 624 (another example of the rotating mechanism), thereby realizing the rotation of the bed surface around the Y axis.

The process of transferring the user from the bed function module to the movement transfer function module through the cooperative work of the bed function module and the movement transfer function module is as follows:
1. The user issues an instruction;
2. The movement transfer function module moves close to the bed function module and is connected to the bed function module through the connection joint;
3. The height of the seat of the movement transfer function module is adjusted to be consistent to the height of the bed surface, so as to ensure the smooth transfer of the user from the bed function module to the movement transfer function module.

Different proposals are provided in subsequent steps depending on the user's situation:
(1) For the user who can sit up and the upper limbs of the user can basically work:
   1. The armrest which is included by the movement transfer function module and is close to the bed function module and the armrest which is included by the bed function module and is on the side close to the movement transfer function module are put down;
   2. The user sits up by himself/herself or the bed surface changes from flat-lying state to half-lying state to assist the user to sit up;
   3. The user is translated (moved horizontally) from the bed function module to the seat of the movement transfer function module;
   4. The armrest which is included by the movement transfer function module and is close to the bed function module is raised up, and if necessary, the guardrail can be lowered in front of the user;
   5. The seat of the movement transfer function module returns to a height suitable for walking/movement;
   6. The movement transfer function module is separated from the bed function module and goes to the destination.
(2) For the user who cannot sit up and the upper limbs are insufficient in function:
   1. Partial supporting plates of the bed are raised to form certain space between the user and the bed surface for the mechanical arms to be inserted;
   2. The mechanical arm of the movement transfer function module is inserted below the user to lift up the user, transfer the user to the top of the movement transfer function module, and place the user in the seat in a sitting posture. In the process, if necessary, a protective mechanism (e.g., the guardrail) is added to ensure safety and the comfort and the safety of the user;
   3. The armrest which is included by the movement transfer function module and is close to the bed is raised up, and if necessary, the guardrail can be placed in front of the user;
   4. The seat of the movement transfer function module returns to a height suitable for walking/movement;
   5. The movement transfer function module is separated from the bed function module and goes to the destination.

The execution of each step above can be realized through human-computer interaction between the user and the device if necessary.

### Fourth Embodiment

For instance, in an embodiment of the present disclosure, the bathing manner of the bath function module is sitting bath, and a main structure of the bath function module includes a sitting bath table, a shower device, a drier device and the connection joint. The sitting bath table can clean a part which is included by the user and is in contact with a surface of the bath table by means of vibration, friction, or the like; armrests are mounted at two sides of the sitting bath table and can ascend or descend; for example, after the armrests are put down, a guardrail is formed in front of the body of the user (in other embodiments, the guardrail may not be formed after the armrests are lowered); the shower device (e.g., a shower head) and the drier device are mounted on an upper side of the sitting bath table; the shower device can adjust the water velocity and the water volume; the drier device can adjust the airflow temperature and the air volume; and the connection joint is mounted on the side of the sitting bath table and is configured to be connected with other function modules.

As shown in FIG. 18 which is a schematic diagram of the bath function module of the intelligent nursing system provided by an embodiment of the present disclosure, the reference signs in the figure are: 3. the second connection joint; 701. a protective armrest; 702. another protective armrest; 703. the shower device; 704. the drier device; 705. the sitting bath table. The second connection joint 3 can be in abutting joint with the movement transfer function module. During bathing, the user sits on the sitting bath table 705, and the protective armrests 701 and 702 on both sides are lowered to form a guardrail in front of the user's body. The shower device 703, which is obliquely disposed on the upper side of the sitting bath table 705, sprays water for showering. While the user takes a shower, the sitting bath table 705 can clean the back and the lower thigh of the user by vibration or rotary brush cleaning. After cleaning, the drier device 704 assists the user in surface wiping and drying by means of blowing. The shower device 703 can adjust the water velocity and the water volume and atomize the water stream. The drier device 704 can adjust the airflow temperature and the air volume, and meanwhile, can stir the wind and adjust the wind direction.

For instance, a control button 700 is mounted at the bath function module and can remotely control the movement of the movement transfer function module, so that the movement function module can run by itself to the position of the bath function module to complete the abutting joint with the bath function module, and the movement function module can release the abutting joint state with the bath function module and run to other positions.

The process of transferring the user from the movement transfer function module to the bath function module through the cooperative work of the movement transfer function module and the bath function module is as follows:
1. The user issues an instruction;
2. The movement transfer function module moves close to the bath function module and is connected to the sitting bath table through the connection joint;
3, The height of the seat of the movement transfer function module is adjusted to be consistent with the height of the sitting bath table.

Two transfer methods are provided in subsequent steps according to user status:
(1) The user can sit up on his own and the upper limbs of the user can basically function:
   1. The armrest which is included by the movement transfer function module and is close to the side of the sitting bath table is put down;
   2. The user translates from the movement transfer function module to the sitting bath table, and the movement transfer function module moves to the outside of the bathroom;
   3. The armrests on the two sides of the bath function module are lowered, and a guardrail is formed in front of the user's body;
   4. After bathing, the user issues an instruction, and the movement transfer function module moves close to the bath function module and is connected to the sitting bath table through the connection joint;
   5. The armrests on the two sides of the bath function module are raised, and the user transfers from the sitting bath table to the movement transfer function module;
   6. The armrest which is included by the movement transfer function module and is on the side close to the sitting bath table is raised up, and if necessary, the guardrail can be lowered in front of the user;
   7. The movement transfer function module returns to a height suitable for walking/movement;
   8. The movement transfer function module is separated from the bath function module and goes to the next destination.
(2) The user cannot sit up on his/her own and the upper limbs of the user are insufficient in function:
   1. The armrest which is included by the movement transfer function module and is on the side close to the sitting bath table is put down;
   2. Two mechanical arms of the movement transfer function module protrude; one mechanical arm is on the back of the user, and the guiderail protrudes and surrounds the chest and the underarm of the user to form protection; the other mechanical arm is in the lower limb part of the user, and lifts up the user and transfers the user to the sitting bath table; and the movement transfer function module moves to the outside of the bathroom;
   3. The armrests on the two sides of the bath function module are lowered to form a guardrail in front of the user's body;
   4. After bathing, the user issues an instruction, and the movement transfer function module is connected to the sitting bath table through the connection joint when approaching the bath function module;
   5. The armrests on the two sides of the bath function module are raised up, and two mechanical arms of the movement transfer function module protrude and move the user to the seat of the movement transfer function module according to the foregoing steps;
   6. The armrest which is included by the movement transfer function module and is on the side close to the sitting bath table is raised up, and if necessary, the guardrail can be lowered in front of the user;
   7. The movement transfer function module returns to a height suitable for walking/movement;
   8. The movement transfer function module is separated from the bath function module and goes to the next destination.

The execution of each step above can be realized through human-computer interaction between the user and the device if necessary.

### Fifth Embodiment

For instance, in an embodiment of the present disclosure, the connection joints includes the first connection joint and the second connection joint. The first connection joint and the second connection joint can be respectively mounted at two function modules and can also be that: one connection joint is a connection joint with a fixed mounting position (for instance, the one connection joint is mounted at an object with fixed a position such as a floor or a wall surface or the like), and the other connection joint is mounted at the function module. The mechanical abutting joint between two function modules is realized through the abutting joint between the first connection joint and the second connection joint. For instance, the mechanical abutting joint and the data transmission between two function modules can also be realized through the abutting joint of two joints.

As shown in (a) and (b) in FIG. 19 which are schematic diagrams of the intelligent nursing system provided by an embodiment of the present disclosure before and after the abutting joint of the connection joints, the reference signs in the figure are: 31. a data signal and electrical signal connection female; 32. a mechanical locking mechanism; 33. another mechanical locking mechanism; 34. a positioning sensor; 35. an electromagnetic connector mechanism; 36. a mechanical guide mechanism; 37. another positioning sensor; 38. a data signal and electrical signal connection male; 39. another mechanical guide mechanism. For instance, as shown in (a) in FIG. 19, each of the first connection joint 401 and the second connection joint 3 includes the mechanical guide mechanism (see 36 and 39) and the positioning sensor (see 37 and 34). One of the first connection joint 401 and the second connection joint 3 further includes the electromagnetic connector mechanism 35 and the data signal and electrical signal connection male 38, and the other one of the first connection joint 401 and the second connection joint 3 further includes mechanical locking mechanisms 32 and 33 and the data signal and electrical signal connection female 31. For instance, 35, 36, 37, 38 form the first connection joint 401, and 31, 32, 33, 34, 39 form the second connection joint 3, as shown in (a) and (b) in FIG. 19; or, 35, 36, 37, 38 form the second connection joint 3, and 31, 32, 33, 34, 39 form the first connection joint 401.

The combination of the connection joints includes a connection joint guide device, a rigid connector device and a data transmitter device, and the connection joint guide device, the rigid connector device and the data transmitter device respectively include different components of the first connection joint and the second connection joint.

The connection joint guide device includes the mechanical guide mechanisms (see 36 and 39) and the positioning sensors (see 37 and 34) of the first connection joint 401 and the second connection joint 3. The connection joint guide device relies on the positioning sensors to guide the two function modules that need to be abutted with each other to adjust an abutting-joint position at a long distance, and relies on the mechanical guide mechanisms to adjust a position error between the two function modules that need to be abutted with each other at a close distance.

The rigid connector device includes the electromagnetic connector mechanism 35 of one of the first connection joint 401 and the second connection joint 3, and includes the mechanical locking mechanisms 32 and 33 of the other one of the first connection joint 401 and the second connection joint 3. The rigid connector device can rely on the electromagnetic connector mechanism to realize the rigid connection between two connection joints of the two function modules, and rely on the mechanical locking mechanisms to enhance connection. The mechanical locking mechanisms can be locked automatically or manually.

The data transmitter device includes the data signal and electrical signal connection male 38 and the data signal and electrical signal connection female 31. The connection and fitting between the data signal and electrical signal connection male and the data signal and electrical signal connection female of the data transmitter device are completed during the abutting joint of the first connection joint and the second connection joint to form a data transmission channel between the two function modules.

When the first connection joint 401 and the second connection joint 3 are butted, the abutting joint orientation is determined by the positioning sensors 34 and 37 at first, thereby guiding the two function modules to approach each other from mutually parallel directions. When the distance between the two function modules is close to a certain position to bring the two mechanical guide mechanisms 36 and 39 into contact, the first connection joint 401 continues to move with the movement transfer function module under the guidance of the mechanical guide mechanism until it moves to a set abutting joint position. At this time, the data signal and electrical signal connection male 38 and the data signal and electrical signal connection female 31 are connected to each other; the electromagnetic connector mechanism 35 is started; mechanical locking by the mechanical locking mechanisms 32 and 33 is completed; a detection system detects that the abutting joint process is completed; and the combination of the function modules can carry out the next task.

### Sixth Embodiment

The embodiment of the present disclosure provides the seat function module. For instance, as shown in FIG. 20, the seat function module includes a seat 20 and the connection joint 3 connected with the seat 20. For instance, the seat 20 may be a sofa, a chair or other types of seat.

The seat function module can be connected with the movement transfer function module or other function module through the connection joint 3. For instance, arrangement of the connection joint 3 may refer to relevant description in the embodiment 5, and no further description will be given here. For instance, the seat function module may further include an armrest which is configured to ascend or descend or form a guiderail. The movement manner of the user between the movement transfer function module and the seat function module may refer to relevant description in the embodiments 2-4, and no further description will be given here.

### Seventh Embodiment

The embodiment of the present disclosure provides the rehabilitation exercise function module. For example, as shown in FIG. 21, the rehabilitation exercise function module includes a hip plate 856, a leg plate 816, a roller 817, a cam 818, and the connection joint 3. The cam 818 drives the leg plate 816 to move through the roller 817 during rotation of the cam 818, so that the leg plate 816 can swing around the junction of the leg plate 816 and the hip plate 856 to assist the user in leg rehabilitation exercise when the user sits on the hip plate 856. For example, the cam 818 can be fixed below the hip plate 856. For example, the rehabilitation exercise function module may further include a back plate 815. By adjusting the inclination angle of the back plate 815, the user can realize the conversion among the sitting posture, the upper body flat-lying posture and the upper body half-lying posture.

The rehabilitation exercise function module can be connected with the movement transfer function module or other function module through the connection joint 3. For instance, arrangement of the connection joint 3 may refer to relevant description in the fifth embodiment, and no further description will be given here. For instance, the rehabilitation exercise function module may further include an armrest which is configured to ascend or descend or form a guiderail. The movement manner of the user between the movement transfer function module and the rehabilitation exercise function module may refer to relevant description in the embodiments 2-4, and no further description will be given here.

### Eighth Embodiment

At least one embodiment of the present disclosure provides another intelligent nursing system. As shown in FIG. 22, the intelligent nursing system includes a nursing function module and the movement function module 4. The nursing function module includes at least one selected from the group consisting of the bed function module 6, the toilet bowl function module 5, the bath function module 7, the seat function module 2 and the rehabilitation exercise function module 9. The movement function module 4 is configured to carry the user and move to the nursing function module. In the intelligent nursing system, each of the movement function module 4 and the nursing function module includes a connection joint (as shown by 401 and 3 in FIG. 22), and the connection joints in the movement function module 4 and the nursing function module can be rigidly connected with each other, that is to say, in the case that the connection joints are rigidly connected with each other, when one of the connection joints is displaced, the other connection joint connected to the one connection joint is not displaced relative to the one connection joint.

In the embodiment of the present disclosure, because the connection joints are respectively fixed on respective function modules and are rigidly connected with each other, the relative displacement between the function modules can be prevented, so that the safe and stable movement of the user can be guaranteed.

For instance, the movement function module 4 may also be configured to move the user from the position of the movement function module to the position of the nursing function module, or move from the position of the nursing function module to the position of the movement function module. For instance, the movement function module 4 may include a mechanical arm.

The movement function module in the embodiment of the present disclosure may refer to the description on the movement transfer function module in the above embodiments. The nursing function module and the connection joint in the embodiment of the present disclosure may refer to the related description in the above embodiment, and no further description will be given here.

Based on the above technical proposals, the embodiments of the present disclosure can realize the function of performing at least one care action on the daily life of the elderly or the inconvenient person under the condition of unattended care. Specifically, the advantages of the embodiments of the present disclosure are as follow. (1) The embodiments of the present disclosure adopt a modular design concept, are aimed at main activities of the elderly or the inconvenient person in unattended conditions, e.g., toilet, bathing and rehabilitation exercise, provide specific implementation plans, and provide feasible technical means to help the elderly or the inconvenient person to achieve self-care in daily life, so that the increasingly serious problem of old-age care can be free from the high dependence on nursing staff. (2) The modules used in the embodiments of the present disclosure not only can independently realize respective functions but also can be rigidly connected with each other through the connection joints to realize cooperative work; the intelligent nursing system provided by the embodiments of the disclosure is convenient and efficient and realizes the comprehensive coverage of various items in daily life; as for the special needs of different groups of people, it only needs to add corresponding function modules. (3) The way of connecting different function modules through the connection joints ensures the safety and the comfort of the user when moving between different function modules.

What are described above is related to the illustrative embodiments of the disclosure only and not limitative to the scope of the disclosure; the scopes of the disclosure are defined by the accompanying claims.

This application claims the benefit of Chinese patent application No. 201611204964.3 filed on December 23, 2016, which is hereby entirely incorporated by reference as a part of the present application.

## Claims

1. An intelligent nursing system, comprising:
a movement transfer function module; and
at least one selected from the group consisting of a bed function module, a toilet bowl function module, a bath function module, a seat function module and a rehabilitation exercise function module,
wherein each function module among the movement transfer function module, the bed function module, the toilet bowl function module, the bath function module, the seat function module and the rehabilitation exercise function module comprises a connection joint; and in a situation where two or more than two function modules are configured to cooperate with each other, the two or more than two function modules are configured to be connected with each other through the connection joints of the two or more than two function modules.

2. The intelligent nursing system according to claim 1, wherein the connection joint of each of the bed function module, the toilet bowl function module, the bath function module, the seat function module and the rehabilitation exercise function module and the connection joint of the movement transfer function module are configured to be rigidly connected with each other.

3. The intelligent nursing system according to claim 1 or 2, wherein the connection joints are configured to implement data transmission between the two or more than two function modules after connection.

4. The intelligent nursing system according to any one of claims 1 to 3, wherein different function modules are distributed in different activity spaces, and the movement transfer function module is configured to transport a user to move in the different activity spaces.

5. The intelligent nursing system according to any one of claims 1 to 4, wherein
the movement transfer function module is configured to transport a user to a specified destination, and is configured to help the user to transfer from one function module to another function module.

6. The intelligent nursing system according to claim 5, wherein
the movement transfer function module is configured to help the user to realize a conversion between a sitting posture and a standing posture; and/or
the movement transfer function module is configured to be used in cooperation with a vehicle.

7. The intelligent nursing system according to any one of claims 1 to 6, wherein
the movement transfer function module comprises a movement mechanism and a riding mechanism;
the riding mechanism comprises a multi-stage ascending-descending device, a cushion, armrests, a backrest and a pedal;
the movement mechanism comprises a chassis, a drive wheel, a driven wheel and a driven wheel slide rail, the drive wheel is mounted at the chassis, and the driven wheel is connected with the chassis through the driven wheel slide rail;
the multi-stage ascending-descending device is mounted on the chassis;
the cushion is mounted on an upper side of the multi-stage ascending-descending device;
the backrest is placed on the upper side of the cushion;
the armrests and the pedal are configured to be foldable;
in a situation where both the armrests and the pedal are in an unfolded state, the armrests are on two sides of the riding mechanism respectively, and the pedal is in front of the chassis and is higher than an upper surface of the chassis.

8. The intelligent nursing system according to claim 7, wherein
the multi-stage ascending-descending device comprises a cushion support rod, a support frame, an ascending-descending pillar that is configured to ascend or descend in a vertical direction, and an obliquely ascending-descending mechanism that is configured to ascend or descend in an oblique upward direction;
the obliquely ascending-descending mechanism comprises an oblique support frame, a guide rail, a slider and a push rod;
the slider is connected with the cushion support rod;
the support frame and the obliquely ascending-descending mechanism are configured to be driven by the ascending-descending pillar to ascend or descend in the vertical direction;
both the armrests and the backrest are connected with the support frame and configured to be driven by the ascending-descending pillar to ascend or descend together with the cushion;
the push rod is configured to push the slider to move obliquely upward along the guide rail while driving the cushion to move obliquely upward, so as to achieve a function of assisting a user to stand.

9. The intelligent nursing system according to claim 7 or 8, wherein
a minimum distance from an upper surface of the cushion to the ground during movement is not greater than 400 mm; and
a ratio of a maximum distance, which is from the upper surface of the cushion to the ground during movement, to the minimum distance is not greater than 2.5.

10. The intelligent nursing system according to any one of claims 7 to 9, wherein the drive wheel comprises a single wheel for drive or comprises two wheels which are configured to be driven independently, and/or the drive wheel comprises an electromagnetic brake device.

11. The intelligent nursing system according to any one of claims 7 to 10, wherein the movement mechanism comprises a plurality of driven wheels; and the plurality of driven wheels are configured to have a driven wheel track which is changeable during movement of the movement transfer function module.

12. The intelligent nursing system according to claim 11, wherein
the movement mechanism further comprises a driven wheel track changing mechanism;
the driven wheel track changing mechanism comprises the driven wheel slide rail, a slider, a connecting rod, a rotating disc and a motor;
the plurality of driven wheels are fixed at the driven wheel slide rail;
the driven wheel slide rail is connected with the rotating disc through the connecting rod; and
in a situation where the rotating disc is driven by the motor to rotate around a central axis, the connecting rod pushes the driven wheel slide rail to contract or extend along a direction of the slider to change the driven wheel track.

13. The intelligent nursing system according to any one of claims 7 to 12, wherein
in a situation where the movement transfer function module is under a non-manned state, the armrests and the pedal are configured to be folded to inside of the movement transfer function module, and the backrest is configured to be folded to be on the upper side of the cushion; and
in a situation where the movement transfer function module works under a manned movement state, the backrest, the armrests and the pedal are configured to be in an unfolded state and are disposed at respective functional positions.

14. The intelligent nursing system according to claim 13, wherein the movement transfer function module is configured to perform a non-manned movement, is battery-driven, and is configured to start an automatic charging program based on a detection of the intelligent nursing system that electric quantity of the movement transfer function module is reduced to be below a threshold.

15. The intelligent nursing system according to claim 13 or 14, wherein in the situation where the movement transfer function module works under the manned movement state, the driven wheel is configured to protrude from a position below the chassis to increase a driven wheel track.

16. The intelligent nursing system according to any one of claims 13 to 15, wherein
the riding mechanism further comprises cushion plates;
in a situation where the movement transfer function module works under a state of assisting a user to stand, the pedal is folded up, the cushion ascends upward and extends obliquely upward under cooperative work of the multi-stage ascending-descending device, and the cushion plates on two sides of a front end of the cushion are folded downward to narrow the front end of the cushion, so that a posture of the user changes from sitting to riding.

17. The intelligent nursing system according to any one of claims 7 to 16, wherein
a movement manner of the movement transfer function module comprises a manual control manner and/or an automatic control manner;
the movement transfer function module comprises a control handle and is configured to adopt the control handle to control under the manual control manner, and the control handle is mounted on the armrest on one of the two sides;
under the automatic control manner, the movement transfer function module is configured to implement path guidance through an automatic navigator device mounted on the movement transfer function module, the movement transfer function module is provided with a wireless data transmission interface to execute commands by means of wireless remote control, the movement transfer function module is provided with an obstacle avoidance sensor to achieve a function of stop or bypass in presence of encountering an obstacle, the movement transfer function module is provided with a voice interaction device to provide a voice prompt before or after abutting joint and/or transfer action, and the voice interaction device has functions of voice recognition and voice positioning and is configured to determine a user's position based on voice information sent by the user.

18. The intelligent nursing system according to claim 17, wherein
the movement transfer function module further comprises a master-control chip;
a mounting plate is disposed on an upper side of the chassis;
the automatic navigator device, the wireless data transmission interface, the obstacle avoidance sensor, the voice interaction device and the master-control chip are mounted at the mounting plate; and
the control handle, the automatic navigator device, the wireless data transmission interface, the obstacle avoidance sensor and the voice interaction device are respectively connected with the master-control chip.

19. The intelligent nursing system according to any one of claims 7 to 18, wherein
an illuminator device is mounted in front of the riding mechanism of the movement transfer function module, and/or
the automatic navigator device adopt a magnetic navigator, a laser navigator or an inertial navigator.

20. The intelligent nursing system according to any one of claims 1 to 19, wherein
the bed function module comprises a bed surface, a supporting plate, armrests and baffle plates;
the supporting plate is mounted in a middle portion of the bed surface;
the armrests are on two sides of the bed surface, respectively;
the baffle plates are at a head end and a tail end of the bed surface, respectively; and
each of the armrests and the baffle plates is configured to be adjustable in height and be lowered to be below a plane where the bed surface is located.

21. The intelligent nursing system according to claim 20, wherein
the bed surface is in a split joint form and comprises a back plate, a hip plate and a leg plate;
the back plate, the hip plate and the leg plate of the bed surface are configured to be adjustable in height and angle; and
the bed surface is configured to allow a user to change postures among a flat-lying posture, a half-lying posture and a sitting posture.

22. The intelligent nursing system according to claim 20 or 21, wherein the bed surface is configured to be adjustable in height.

23. The intelligent nursing system according to any one of claims 20 to 22, wherein
the bed function module comprises a plurality of supporting plates arranged side by side;
the plurality of supporting plates are configured to be respectively disposed below a back, a hip and a leg of a user in a situation where the user is in a flat-lying posture; and
at least partial of the plurality of supporting plates of the bed function module are configured to be able to ascend or descend.

24. The intelligent nursing system according to any one of claims 20 to 23, wherein
control buttons are disposed at end portions of the head end and the tail end of the bed function module and configured to control motions of components of the bed function module; and/or
the bed function module is provided with a data transmission interface which is configured to be connected with a smart machine for remote control of a motion of the bed function module.

25. The intelligent nursing system according to any one of claims 20 to 24, wherein
the bed function module comprises two rotation axes which mutually form 90°;
one axis of the two rotation axes is configured to be parallel to a body direction of a user in a flat-lying posture, and the other axis of the two rotation axes is configured to be perpendicular to the body direction of the user in the flat-lying posture;
the bed surface and the two rotation axes are connected with each other through a rotating mechanism; and
the bed surface is configured to integrally rotate around each of the two rotation axes.

26. The intelligent nursing system according to any one of claims 1 to 25, wherein
the toilet bowl function module comprises a toilet bowl, an auxiliary heightening cushion, a cleaning and drier device, toilet bowl armrests and an auxiliary trouser removal device;
the auxiliary heightening cushion is mounted on an upper side of the toilet bowl and configured to adjust a hip height of a user in use of the toilet bowl;
the toilet bowl armrests are disposed on two sides of the toilet bowl and configured to be foldable and be able to ascend or descend;
the auxiliary trouser removal device is disposed beside the toilet bowl, a main structure of the auxiliary trouser removal device comprises auxiliary trouser removal mechanical arms which are respectively disposed on the two sides of the toilet bowl, and end portions of the auxiliary trouser removal mechanical arms are configured to be connected with a waist of trousers of a user and drag the waist of trousers down or up along a thigh direction to finish trouser removal or wearing action; and
the cleaning and drier device is configured to help the user to clean and dry after use.

27. The intelligent nursing system according to claim 26, wherein
the auxiliary heightening cushion has a cushion ring heating function; and/or
the auxiliary trouser removal device further comprises pulling force sensors disposed at the auxiliary trouser removal mechanism arms, and the auxiliary trouser removal mechanism arms are configured to stop motion based on a detection of the pulling force sensors that a pulling force exceeds a certain value.

28. The intelligent nursing system according to any one of claims 1 to 27, wherein a bathing manner of the bath function module is sitting bath, and the bath function module comprises a sitting bath table, a shower device and a drier device;
the sitting bath table is configured to clean a part which is comprised by a user and is in contact with a surface of the sitting bath table, and armrests are mounted on two sides of the sitting bath table and configured to be able to ascend or descend;
the shower device and the drier device are mounted on an upper side of the sitting bath table;
the shower device is configured to adjust a water velocity and a water volume; and
the drier device is configured to adjust an airflow temperature and an air volume.

29. The intelligent nursing system according to claim 28, wherein the bath function module is provided with a control button which is configured to remotely control a movement of the movement transfer function module, so that the movement transfer function module automatically runs to a position of the bath function module and completes abutting joint with the bath function module.

30. The intelligent nursing system according to claim 28 or 29, wherein the armrests are configured to form a guardrail in front of the user's body after being lowered.

31. The intelligent nursing system according to any one of claims 1 to 30, wherein
the connection joints comprises a first connection joint and a second connection joint;
the first connection joint and the second connection joint are respectively mounted at two function modules among the two or more than two function modules; or one of the first connection joint and the second connection joint is a connection joint with a fixed mounting position, and the other of the first connection joint and the second connection joint is mounted at one of the two or more than two function modules; and
a mechanical abutting joint between two function modules among the two or more than two function modules is realized by abutting joint between the first connection joint and the second connection joint.

32. The intelligent nursing system according to claim 31, wherein
the connection joint comprises a connection joint guide device, a rigid connector device and a data transmitter device;
each of the first connection joint and the second connection joint comprises a mechanical guide mechanism and a positioning sensor, one of the first connection joint and the second connection joint further comprises an electromagnetic connector mechanism and a data signal and electrical signal connection male, and the other of the first connection joint and the second connection joint further comprises a mechanical locking mechanism and a data signal and electrical signal connection female;
the connection joint guide device comprises the mechanism guide mechanisms and the positioning sensors of the first connection joint and the second connection joint;
the rigid connector device comprises the electromagnetic connector mechanism and the mechanical locking mechanism; and
the data transmitter device comprises the data signal and electrical signal connection male and the data signal and electrical signal connection female.

33. The intelligent nursing system according to any one of claims 1 to 32, wherein a working voltage of each component of each function module does not exceed 36V during a use process of the intelligent nursing system by a user.

34. The intelligent nursing system according to any one of claims 1 to 33, wherein
the rehabilitation exercise function module comprises a hip plate, a leg plate, a roller and a cam; and
the cam is configured to drive the leg plate to move through the roller during rotation of the cam, so that the leg plate swings around a junction of the leg plate and the hip plate.

35. An intelligent nursing system, comprising:
a nursing function module comprising at least one selected from the group consisting of a bed function module, a toilet bowl function module, a bath function module, a seat function module and a rehabilitation exercise function module; and
a movement function module configured to move to the nursing function module,
wherein each of the nursing function module and the movement function module comprises a connection joint, and the connection joints of the nursing function module and the movement function module are configured to be rigidly connected with each other.

36. The intelligent nursing system according to claim 35, wherein the movement function module is further configured to allow a user to move from a position of the movement function module to a position of the nursing function module, or move from the position of the nursing function module to the position of the movement function module.
